# EUROPEAN PATENT SPECIFICATION

(11) **EP 1 789 375 B1**
(45) Date of publication and mention of the grant of the patent: **09.12.2015**
(21) Application number: 05787232.7
(22) Date of filing: 26.08.2005
(51) Int. Cl.: C07C 51/08, C07C 51/10, C07C 57/58

(54) **PROCESS FOR THE PRODUCTION OF [2-(4-FLUORO-BENZYL)-PHENYL]-ACETIC ACID**
VERFAHREN ZUR HERSTELLUNG VON [2-(4-FLUOR-BENZYL)-]PHENYL]-ESSIGSÄURE
PROCEDE DE PRODUCTION D'ACIDE [2- (4-FLUORO-BENZYL-) PHENYL]-ACETIQUE

(30) Priority: 31.08.2004 WO PCT/EP2004/051969
(43) Date of publication of application: 30.05.2007
(73) Proprietor: Janssen Pharmaceutica NV, 2340 Beerse (BE)
(72) Inventor: GUILLAUME, Michel, Joseph, Maurice, André, B-2340 Beerse (BE); LANG, Yolande, Lydia, B-2340 Beerse (BE); ROESSLER, Armin, Cilag AG, Pharmaceutical & Chem., CH-8205 Schaffhausen (CH); ULMER, Lars, 8218 Neuhausen (CH); LEURS, Stefan, Marcel, Herman, B-2340 Beerse (BE); DE SMAELE, Dirk, Janssen Pharmaceutica N.V., B-2340 Beerse (BE)
(74) Representative: Daelemans, Frank F.R.
(86) International application number: PCT/EP2005/054202
(87) International publication number: WO 2006/024630

(56) References cited:
- WO-A-03/048146
- WO-A-03/048147
- US-A1- 2002 062 041

## Description

The present invention relates to a novel process for the production of [2-(4-fluoro-benzyl)-phenyl]-acetic acid, a compound obtainable from phthalic anhydride.

This derivative is an important intermediate in the synthesis of a series of compounds, disclosed in WO 97/38991, having the general formula (A) wherein R¹ and R¹ are *inter alia* hydrogen and/or methyl, R³ and R⁴ are hydrogen or halogen, X is CH₂, O or S, n is 1 and p and q are 0, 1 or 2.

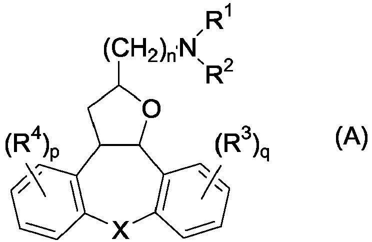

Compounds according to Formula (A) were found useful for the treatment and/or the prevention of CNS disorders, cardiovascular disorders and gastrointestinal disorders. Their synthesis has, among others, been described in WO03/048146 and WO03/048147. In the latter documents (see Scheme 1), it has been disclosed that the [2-(4-fluoro-benzyl)-phenyl]-acetic acid according to Formula (I) can be prepared by adaptation of an art-known sequence (French Patent. No. 4395M, dated October 10, 1966; Can. J. Chem., 1971, 49, 746-754) starting with a Friedel-Crafts acylation reaction using fluorobenzene and phthalic anhydride to form a keto-acid according to Formula (IV), followed by reductive removal of the ketone group and homologation of the carboxylic acid function.

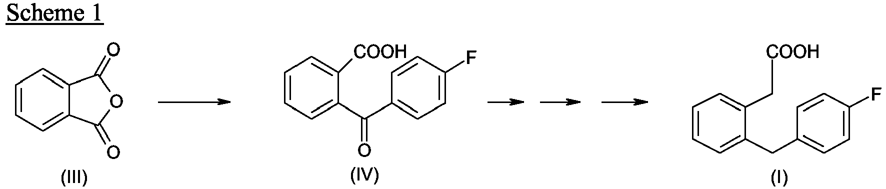

The problem with this reaction scheme is that several steps of the synthesis suffer from diverse drawbacks: environmentally unfriendly solvents, low yield, use of complex reagents or formation of undesired salt mixtures, such as, for example Et₃NH⁺Cl⁻.

The object of the present invention is to provide a process for the production of [2-(4-fluoro-benzyl)-phenyl]-acetic acid which is suitable for industrial scale reactors (e.g. which is cleaner and more efficient).

A further object of the present invention is to provide a process such that [2-(4-fluoro-benzyl)-phenyl]-acetic acid is obtained as a crystalline material with a purity > 95%.

Very surprisingly, the inventors have found that the drawbacks of the known processes can be overcome by a process which comprises the subsequent steps a) through e:
a) reacting phthalic anhydride with fluorobenzene in a Friedel Crafts reaction using fluorobenzene itself as solvent and aluminium chloride as the Lewis acid at reflux temperature, wherein aluminium chloride is used in a molar ratio related to phthalic anhydride >2:1;
b) over reducing the product obtained in step a) at the ketone moiety using hydrogen gas, optionally in the presence of a Pd/C catalyst and using isopropanol (iPrOH) as solvent;
c) reducing the product obtained in step b) with 2.3 equivalents of sodium dihydro-bis (2-methoxyethoxy) aluminate (Red-Al) to the corresponding alcohol;
d) chlorinating the alcohol obtained in step c) using aqueous hydrochloric acid as the chlorinating agent at 90 °C in a closed vessel and
e) inserting CO into the product obtained in step d) through an appropriate Pd-containing catalytic system by reacting with CO in a mixture of THF and water in the presence of a) sodium acetate, K2C03 or Et3N and b) triphenylphosphine.

A similar reaction step e) *per se* is disclosed in EP 1 207 148 A1 (Clariant GmbH, 22 May 2002).

The process according to the invention can schematically be depicted as follows (see Scheme 2):

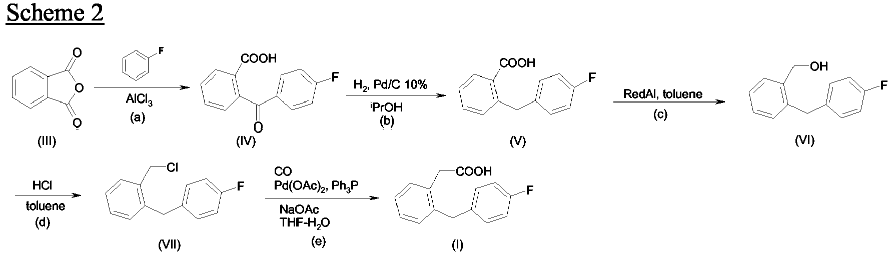

In step a), a Friedel Crafts reaction is used using fluorobenzene itself as solvent and aluminium chloride as the Lewis acid for the Friedel-Crafts reaction. Aluminium chloride is used in a molar ratio related to phthalic anhydride > 2:1. Using less aluminium chloride leads to incomplete conversion. Preferentially, in step a), the reaction is performed at reflux temperature of fluorobenzene, which is 75-80°C. When the reaction is performed at a lower temperature, the reaction rate decreases. Preferentially, the reaction mixture obtained in step a), which includes a number of aluminium salts, is hydrolysed with aqueous hydrochloric acid.

In step b), the reaction is performed using hydrogen gas, optionally in the presence of a Pd/C catalyst and using isopropanol (iPrOH) as solvent. Using methanol (MeOH) instead leads to decreased reaction rate. Using water leads to formation of impurities. Preferentially, in step b), the reaction is performed at a temperature above 45°C. Below 45°C, the reaction is too slow.

In step c), 2.3 equivalents of sodium dihydro-bis (2-methoxyethoxy) aluminate ("RedAl") is used. Using less reducing agent could lead to a lower conversion. Preferentially, in step c), toluene is used as a solvent. Toluene is the solvent in which the commercial sodium dihydro-bis (2-methoxyethoxy) aluminate ("RedAl") is dissolved. Due to the reactive nature of this reductant , it is not possible to use protic solvents. Moreover, it is not necessary to isolate the product obtained in step (c) after work-up because the toluene solution comprising the product is used as it is.

In step d) aqueous hydrochloric acid is used as the chlorinating agent. In step d), the reaction is performed at 90°C. The reaction rate is lower at a lower temperature. In step d), the reaction is performed in a closed vessel (pressure build-up occurs to 151.987 MPa (1.5 atmosphere)). In an open vessel, hydrochloric acid partially evolves.

In step e), the product obtained from step d) is reacted with CO in a mixture of THF and H₂O, preferably in a 1:1 ratio, using a Pd-containing catalytic system. Water is necessary to bring about the hydrolysis of the intermediate palladium complex. Its combination with THF gives the highest conversion. As a catalytic system for this reaction step, preferably sodium acetate is used. If sodium acetate is omitted, hardly any reaction takes place. Other systems comprise potassium carbonate or triethylamine In step e), the reaction is performed using triphenylphosphine (Ph₃P) as a ligand for palladium. Other ligands have been tested, but results are not better. Ph₃P is the more common one and is preferentially used. Preferentially, in step e), the reaction is performed at a pressure of 0.4 MPa (4 bars). At atmospheric pressure, the reaction is very slow. Preferentially, in step e), the reaction is performed at a temperature of 80°C. At a lower temperature, the reaction is slower.

The process according to the invention will now be elucidated using the following examples.

### Experimental

All materials were purchased from commercial suppliers and used without further purification. Reactions were conducted under an atmosphere of nitrogen, when necessary. In the lab, only glass vessels were used; in the pilot plant, both steel or glass-lined vessels are used. For each reaction, a sample of the reaction mixture was collected and analysed by means of HPLC.

### Example

### Step a) 2-(4-fluoro-benzoyl)-benzoic acid (Compound IV)

1. A solution of p-fluorobenzenemagnesium bromide (1.2 M solution in THF, 1 eq.) is added to a 0.4 M solution of phthalic anhydride in THF, so that the temperature remains under 30°C. After 1 hour, half of the solvent is distilled off and the reaction mixture is stirred overnight at room temperature. The obtained precipitate is filtered off and taken up in water (0.3 L/mol). Toluene (1 L/mol) and HCl_{cp} are added so that the temperature remains under 35°C. After stirring for 1 hour, the organic layer is evaporated (50°C, vacuum) and the obtained solid is dried at 50°C under vacuum. Active yield: 69 %.
2. Alternatively, a Friedel-Crafts reaction can be performed. In an inertized flask containing phthalic anhydride, fluorobenzene (1.8 L/mol), is added, followed by cautious addition of aluminium chloride (2.1 eq.). The reaction mixture is heated up to 75°C (HCl evolution is observed). After 3 hours at 75°C, water is added (1 L/mol). The organic layer is separated and concentrated hydrochloric acid (0.1 L/mol) is added. Fluorobenzene is distilled off and the mixture is cooled down to 10°C. The precipitate is filtered off, washed with water and dried at 65-70°C. Active yield: 85 %. N.B. Fluorobenzene can be recuperated by washing with an alkaline solution and discarding the water layer.

### Step b) 2-(4-fluoro-benzyl)-benzoic acid (Compound V)

In a hydrogenation flask was added 2-(4-fluoro-benzoyl)-benzoic acid, propylene glycol monomethyl ether (1 L/mol) and Pd/C 10 % wet (15 g/mol). Hydrogenation was performed at 50°C over 18 hours. Thiophene (0.3 kg/mol) was added and the catalyst was filtered off. The filtrate was heated up to 80°C and water (1.12 L/mol) was added at that temperature. The mixture was heated up to reflux, then cooled down to 25°C and stirred at that temperature during 1 hour. The precipitate was filtered off, washed with water (1 L/mol) and dried at 50°C during 18 hours. Active yield: 83 %.

### Step c) [2-(4-fluoro-benzyl)-phenyl]-methanol (Compound VI)

In an inertized flask containing 2-(4-fluoro-benzyl)-benzoic acid (1 eq.), toluene (0.8 L/mol) is added and the vessel is cooled down to 0-5°C. Sodium dihydro-bis (2-methoxyethoxy) aluminate ("RedAl") (1 M in toluene, 2.3 eq.) is added dropwise and the reaction mixture is stirred at 5°C during 16 hours. Acetone (3 eq.) is added dropwise at 5°C and the mixture is stirred during 15 minutes. The temperature is allowed to rise to 25°C. A sodium hydroxide solution (5 eq.) is added and the mixture is stirred vigorously during 20 minutes. The organic layer is separated and washed again with a slightly acidic aqueous solution. The organic layer is filtered over Dicalite and used further in the next step.

### Step d) [2-(4-fluoro-benzyl)-phenyl]-chloromethane (Compound VII)

In an inertized vessel containing [2-(4-fluoro-benzyl)-phenyl]-methanol solution in toluene, HCl_{cp} (5 eq.) is added, the vessel is closed and the mixture is stirred to 90°C (a pressure of 0.154 MPa (1.54 bar) develops). After 6 hours at 90°C, the reaction mixture is cooled down to 25°C and the vessel is opened. The layers are separated, the organic layer is washed with water, then with slightly alkaline solution and used further in the next step.

### Step e) [2-(4-fluoro-benzyl)-phenyl]-acetic (Compound I)

[2-(4-fluoro-benzyl)-phenyl]-chloromethane (1 eq.), THF (1 L/mol), water (0.7 L/mol), Pd(OAc)₂ (1.3 mol%), dppp (2.6 mol%) and sodium acetate (2.5 eq.) were placed in a inertized reactor. The reaction mixture is placed under a CO pressure of 0.4 MPa (4 bars) and allowed to stir at 80°C for 20 hours. The organic layer is separated and evaporated under pressure. Toluene (0.75 L/mol) is added to the residue and the carboxylic acid is extracted with a 2 N solution of sodium hydroxide (0.75 L/mol). The black particles of palladium are removed by filtration over Celite. The water layer is placed in a flask and acetic acid (0.9 L/mol) is added. The mixture is warmed up to 80°C then allowed to spontaneously cool down. The crystallisation starts around 50°C. The crystals are filtered off at room temperature., washed with water and dried to give black [2-(4-fluoro-benzyl)-phenyl]-acetic acid (91 %). Treatment of 5 g of black [2-(4-fluoro-benzyl)-phenyl]-acetic acid with charcoal in 25 ml of a mixture of acetic acid and water (7/3) allows the isolation of pure white crystals of [2-(4-fluoro-benzyl)-phenyl]-acetic acid. This step can be performed before the first addition of acetic acid.

Every step has been optimised in the lab, then successfully scaled-up in the pilot plant.

## Claims

1. Process for the production of [2-(4-fluoro-benzyl)-phenyl]-acetic acid, comprising the subsequent steps a) through e):
a) reacting phthalic anhydride with fluorobenzene in a Friedel Crafts reaction using fluorobenzene itself as solvent and aluminium chloride as the Lewis acid at reflux temperature, wherein aluminium chloride is used in a molar ratio related to phthalic anhydride >2:1;
b) over reducing the product obtained in step a) at the ketone moiety using hydrogen gas, optionally in the presence of a Pd/C catalyst and using isopropanol (*i*PrOH) as solvent;
c) reducing the product obtained in step b) with 2.3 equivalents of sodium dihydro-bis (2-methoxyethoxy) aluminate (Red-Al) to the corresponding alcohol;
d) chlorinating the alcohol obtained in step c) using aqueous hydrochloric acid as the chlorinating agent at 90 °C in a closed vessel;
e) inserting CO into the product obtained in step d) through an appropriate Pd-containing catalytic system by reacting with CO in a mixture of THF and H₂O in the presence of a) sodium acetate, K₂CO₃ or Et₃N and b) triphenylphosphine

2. The process according to claim 1 wherein in step e) CO is inserted into the product obtained in step d) through an appropriate Pd-containing catalytic system by reacting with CO in a mixture of THF and H₂O in the presence of sodium acetate and triphenylphosphine.

3. The process according to claim 1, wherein in in step e) the mixture of THF and H₂O is in a 1:1 ratio.

## Patentansprüche

1. Verfahren zur Herstellung von [2-(4-Fluorbenzyl)-phenyl]essigsäure, umfassend die nachfolgenden Schritte a) bis e):
a) Umsetzen von Phthalsäureanhydrid mit Fluorbenzol in einer Friedel-Crafts-Reaktion unter Verwendung von Fluorbenzol selbst als Lösungsmittel und Aluminiumchlorid als Lewis-Säure bei Rückflusstemperatur, wobei Aluminiumchlorid in einem Molverhältnis zu Phthalsäureanhydrid > 2:1 verwendet wird;
b) Überreduzieren des in Schritt a) erhaltenen Produkts an der Ketongruppierung unter Verwendung von Wasserstoffgas, gegebenenfalls in Gegenwart eines Pd/C-Katalysators und unter Verwendung von Isopranol (iPrOH) als Lösungsmittel;
c) Reduzieren des in Schritt b) erhaltenen Produkts mit 2,3 Äquivalenten Natriumdihydro-bis(2-methoxyethoxy)aluminat (Red-Al) zu dem entsprechenden Alkohol;
d) Chlorieren des in Schritt c) erhaltenen Alkohols unter Verwendung von Salzsäure als Chlorierungsmittel bei 90°C in einem geschlossenen Gefäß;
e) Insertieren von CO in das in Schritt d) erhaltene Produkt durch ein geeignetes Pd-haltiges Katalysatorsystem durch Umsetzen von CO in einer Mischung von THF und H₂O in Gegenwart von a) Natriumacetat, K₂CO₃ oder Et₃N und b) Triphenylphosphin.

2. Verfahren nach Anspruch 1, bei dem die Insertion von CO in das in Schritt d) erhaltene Produkt in Schritt e) durch ein ein geeignetes Pd-haltiges Katalysatorsystem durch Umsetzen von CO in einer Mischung von THF und H₂O in Gegenwart von Natriumacetat und Triphenylphosphin erfolgt.

3. Verfahren nach Anspruch 1, bei dem in Schritt e) die Mischung von THF und H₂O ein Verhältnis von 1:1 aufweist.

## Revendications

1. Procédé pour la production d'acide [2-(4-fluorobenzyl)phényl]acétique, comprenant les étapes a) à e) suivantes :
a) la réaction d'anhydride phtalique avec du fluorobenzène dans une réaction de Friedel et Crafts à l'aide de fluorobenzène lui-même en tant que solvant et de chlorure d'aluminium en tant qu'acide de Lewis à la température de reflux, le chlorure d'aluminium étant utilisé en un rapport molaire par rapport à l'acide phtalique > 2:1 ;
b) la surréduction du produit obtenu dans l'étape a) au niveau de la fraction cétone à l'aide d'hydrogène gazeux, éventuellement en présence d'un catalyseur Pd/C et à l'aide d'isopropanol (iPrOH) en tant que solvant ;
c) la réduction du produit obtenu dans l'étape b) avec 2,3 équivalents de dihydrobis(2-méthoxyéthoxy)aluminate de sodium (Red-Al) en l'alcool correspondant ;
d) la chloration de l'alcool obtenu dans l' étape c) à l'aide d'acide chlorhydrique en solution aqueuse en tant qu'agent de chloration à 90 °C dans un récipient fermé ;
e) l'insertion de CO dans le produit obtenu dans l'étape d) grâce à un système catalytique contenant du Pd approprié par réaction avec du CO dans un mélange de THF et d'H₂O en présence a) d'acétate de sodium, de K₂CO₃ ou d'Et₃N et b) de triphénylphosphine.

2. Procédé selon la revendication 1 dans lequel dans l'étape e) du CO est inséré dans le produit obtenu dans l'étape d) grâce à un système catalytique contenant du Pd approprié par réaction avec du CO dans un mélange de THF et d'H₂O en présence d'acétate de sodium et de triphénylphosphine.

3. Procédé selon la revendication 1, dans lequel dans l' étape e) le mélange de THF et d'H₂O est en un rapport de 1:1.
